(19) Europäisches Patentamt
European Patent Office
Office européen des brevets

(11) **EP 4 471 784 A1**

(12) **DEMANDE DE BREVET EUROPEEN**

(43) Date de publication:
**04.12.2024 Bulletin 2024/49**

(21) Numéro de dépôt: **23176319.4**

(22) Date de dépôt: **31.05.2023**

(51) Classification Internationale des Brevets (IPC):
**G16B 10/00** (2019.01)    **G16B 40/20** (2019.01)
**G16B 20/20** (2019.01)    **G16B 20/40** (2019.01)
**G16B 30/10** (2019.01)

(52) Classification Coopérative des Brevets (CPC):
**G16B 20/20; G16B 10/00; G16B 20/40;
G16B 30/10; G16B 40/20**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**KH MA MD TN**

(71) Demandeur: **Biomérieux
69280 Marcy l'Étoile (FR)**

(72) Inventeurs:
• **POUSEELE, Hannes
  8400 Oostende (BE)**
• **TETREAU, Guillaume
  38240 Meylan (FR)**
• **CARRIÇO, Joao Andre
  2650-074 Amadora (PT)**

(74) Mandataire: **bioMérieux PI Groupement
mandataires
bioMérieux
69280 Marcy l'Etoile (FR)**

(54)    **PROCEDE ET SYSTEME DE TYPAGE PROBABILISTE DE SOUCHES MICROBIENNES**

(57)    Un procédé de typage microbiologique comprend la fourniture d'une base de données de profils génétiques de souches microbiennes, un arbre phylogénétique, des assignations des profils génétiques à des positions dans l'arbre phylogénétique, et des fréquences de variation des profils dans l'arbre. Le typage d'un microorganisme comprend la mesure du profil génétique du microorganisme, la détermination d'une probabilité de variation de chaque profil génétique de la base de données au profil génétique mesuré, ladite probabilité de variation étant calculée en fonction des fréquences de variation, la détermination de l'appartenance du microorganisme à un taxon de l'arbre si au moins la probabilité de variation pour le taxon est supérieure à un seuil. Selon l'invention, l'arbre phylogénétique est construit à partir d'un core génome de l'espèce du microorganisme, et l'ensemble prédéfini de marqueurs génétiques est choisi dans un génome accessoire de l'espèce du microorganisme.

| 10 | Constitution ou complétion d'une base de données de génomes complets de souches bactériennes |
| 20 | Phase d'apprentissage d'un arbre phylogénétique et de fréquences de variation d'un profil génétique |
| 30 | Phase de prédiction : mesure du profil génétique d'une souche à tester et assignation de la souche à au moins un taxon en fonction des fréquences de variation et du profil mesuré |
| 40 | Mise en œuvre de mesures épidémiologiques |

**FIG. 1**

**Description**

**DOMAINE DE L'INVENTION**

L'invention a trait au domaine du typage génomique de microorganisme, à savoir la caractérisation de souches bactériennes à un niveau taxonomique inférieur à l'espèce en se basant sur leurs génomes. L'invention trouve particulièrement application dans le suivi épidémiologique de souches bactériennes, le suivi de contamination par des souches bactériennes et la recherche des causes racines à des contamination et/ou infection bactérienne, que leur origine soit industrielle, environnementale, vétérinaire, clinique ou autre.

**ETAT DE LA TECHNIQUE**

Le typage génomique de microorganismes, par exemple de bactéries, regroupe l'ensemble des techniques de comparaison de génomes de souches bactériennes dans le but de caractériser ces dernières à des niveaux inférieurs à celui de l'espèce. Le typage permet notamment de déterminer le degré de similarité de deux souches bactériennes entre elles ou encore de classer les souches parmi des subdivisions du niveau espèce, par exemple des groupes clonaux ou des groupes définis par des caractéristiques phénotypiques des bactéries telles que leurs sérotypes ou leur susceptibilité à des antibiotiques.

Les techniques de typage peuvent être classées en deux catégories, les premières se fondant sur l'analyse d'une portion ciblée très restreinte du génome bactérien, tel que le typage à base de marqueurs MLST (pour « MultiLocus Séquence Typing ») dit "16S rRNA", et les secondes se fondant sur la comparaison des génomes obtenus par un séquençage complet (ou WGS pour « Whole Genome Sequencing »), par exemple un typage de type cg-MLST (pour "core genome" MLST"). Si les secondes présentent le plus haut niveau de précision, l'emploi du séquençage complet a de nombreux désavantages au rang desquels la durée, le coût, ou encore la nécessité de mettre en oeuvre des traitements bioinformatiques complexes. *A contrario,* lorsque les premières techniques utilisent un nombre très limité de marqueurs, elles permettent l'emploi de plateformes d'amplification par réaction de polymérase en chaine (ou "PCR") pour leur détection, beaucoup plus rapides et moins coûteuses.

Quelle que soit la technique employée, les typages génomiques de l'état de la technique se fondent sur la recherche et l'utilisation de portions conservées du génome pour caractériser les microorganismes. Par exemple, le typage MLST se fonde sur des gènes de ménage qui présentent une forte stabilité dans le temps, de même que le typage cg-MSLT vise directement la totalité du génome de base (ou "génome coeur" ou "core genome" en anglais, ces trois expressions étant utilisées de manière interchangeable). On observe dans ce type d'approches que l'obtention d'une grande précision nécessite un nombre très conséquent de marqueurs. A titre d'exemple, afin de détecter des groupes clonaux de l'espèce *P. aeruginosa* avec une précision supérieure à 90%, un typage cg-MLST implique plusieurs milliers de marqueurs, rendant *de facto* l'utilisation de plateformes PCR classiques non pertinente, voire impossible.

En résumé, un utilisateur souhaitant réaliser un typage moléculaire, par exemple à des fins de suivi de contaminations microbiennes, doit choisir entre précision et rapidité.

**EXPOSE DE L'INVENTION**

Le but de la présente invention est de proposer un typage moléculaire utilisant un nombre limité de marqueurs génétiques et de grande précision.

A cette fin, l'invention a pour objet un procédé *in vitro* d'identification d'un microorganisme compris dans un échantillon, comprenant :

    a. la fourniture de moyens de stockages informatiques comprenant :

- une base de données de profils génétiques de souches microbiennes appartenant à l'espèce microbienne dudit microorganisme, lesdits profils consistant en la présence ou l'absence d'un ensemble prédéfini de marqueurs génétiques ;
- un arbre phylogénétique de l'espèce dudit microorganisme, des assignations des profils génétiques de la base de données à des positions dans l'arbre phylogénétique, de fréquences de variation des profils génétiques des souches microbiennes de la base de données pour des niveaux prédéfinis de l'arbre phylogénétique ;

    b. la mesure du profil génétique du microorganisme ;
    c. la détermination, mise en oeuvre par ordinateur, pour au moins un niveau de l'ensemble de niveaux prédéfinis de l'arbre phylogénétique :

- d'une probabilité de variation de chaque profil génétique de la base de données appartenant audit niveau par rapport au profil génétique mesuré, ladite probabilité de variation étant calculée en fonction des fréquences de variation audit niveau ;
- de l'appartenance du microorganisme à une subdivision dudit niveau si un premier critère de performance d'identification est atteint pour ladite subdivision, ledit premier critère étant atteint si au moins une probabilité de variation pour ladite subdivision est supérieure à un premier seuil prédéfini.

procédé dans lequel:

- l'arbre phylogénétique est construit à partir d'un core génome de l'espèce du microorganisme ; et
- l'ensemble prédéfini de marqueurs génétiques est choisi dans un génome accessoire de l'espèce du microorganisme.

L'invention repose sur le principe selon lequel les fréquences de variation du génome accessoire, lorsqu'elles sont structurées par un arbre phylogénétique construit sur le génome de base, explique efficacement la diversité d'une espèce microbienne. Faisant application de ce principe, l'invention consiste à construire un "capteur" génomique précis et flexible de cette information pour en faire un usage simple, rapide et fiable pour l'utilisateur. Ce capteur mesure le profil d'une souche à tester et estime sa probabilité qu'il appartienne à un taxon de l'arbre en fonction des fréquences de variation du profil de ce taxon.

L'invention permet ainsi une précision de classification dans plusieurs centaines de subdivisions de l'espèce. A titre d'exemple, il peut être obtenu un taux d'assignation dans un arbre à 18 niveaux supérieur à 80% avec un intervalle de confiance de 95% chez les *Listeria monocytogènes* et les Salmonella avec 23 et 13 marqueurs respectivement. Ce nombre très faible de cibles génétiques permet l'emploi de plateformes d'amplification telles que les plateformes PCR, par exemple les plateformes GENE-UP® et FilmArray commercialisées par la Demanderesse, ou des plateformes d'amplification isothermale, ou encore l'emploi de puces à ADN.

Selon un mode de réalisation, si le premier critère de performance d'identification audit niveau n'est pas atteint alors l'étape c) est mise en oeuvre pour le niveau directement supérieur dans l'arbre phylogénique.

Selon un mode un mode de le nombre de marqueurs des profils génétiques est inférieur à 50, et de préférence compris entre 16 et 30.

Selon un mode de réalisation :

- l'arbre phylogénétique est construit par l'application d'un clustering basé sur des distances génétiques;
- les distances génétiques décroissent depuis la racine vers les feuilles de l'arbre de sorte à définir un niveau dans l'arbre comprenant entre 150 et 350 différences dans le core génome.

Selon un mode de réalisation, l'ensemble des marqueurs prédéfinis est sélectionné de manière à minimiser une entropie ou critère d'impureté dudit ensemble à un niveau de l'arbre phylogénique. En particulier, les marqueurs des profils génétiques sont sélectionnés de manière à minimiser une entropie ou un critère d'impureté au niveau comprenant entre 150 et 350 différences dans le core génome.

Selon un mode de réalisation, le premier critère de performance d'indentification comprend en outre l'écart entre la probabilité de variation pour la subdivision et les probabilités de variation pour les autres subdivisions dudit niveau, le premier critère de performance d'identification étant atteint si ledit écart est supérieur à un second seuil prédéfini. En particulier, le second critère de performance d'identification comprend en outre l'écart entre la probabilité de variation pour ledit groupe et les probabilités de variation pour les autres groupes, le second critère de performance d'identification étant atteint si ledit écart est supérieur à un quatrième seuil prédéfini.

Selon un mode de réalisation, la base de données comprend des couples de premier et second seuils, et pour chacun desdits seuils au moins un indicateur de performance d'identification, ledit indicateur de performance comprenant au moins un indicateur lié à la spécificité et/ou à la sensibilité du procédé d'identification. En particulier, l'indicateur de performance d'identification comprend un taux d'assignation correct et/ou une précision brute et/ou une précision balancée. Avantageusement, les indicateurs de performance pour les couples de premier et second seuils sont affichés sur un écran, dans lequel un utilisateur sélectionne une valeur d'indicateur de performance d'identification affichée, et dans lequel le procédé fixe les premier et second seuils à leurs valeurs correspondant à la valeur d'indicateur de performance sélectionnée.

Selon un mode de réalisation, la base de données comprend pour une partie au moins des profils génétiques, l'appartenance desdits profils génétiques à des groupes microbiens distincts, lesdits groupes distincts étant absents de l'arbre phylogénétique, le procédé comportant la détermination, mise en oeuvre par ordinateur :

- de probabilités de variation des profils génétiques appartenant auxdits groupes par rapport au profil génétique mesuré, lesdites probabilités de variation étant calculées en fonction des fréquences de variation à un niveau prédéfini de l'arbre phylogénétique ;

- de l'appartenance du microorganisme à l'un des groupes de microorganisme si subdivision dudit niveau si un second critère de performance d'identification est atteint pour ledit groupe, ledit second critère étant atteint si au moins une probabilité de variation pour ledit groupe est supérieure à un troisième seuil prédéfini.

En particulier, le second critère de performance d'identification comprend en outre l'écart entre la probabilité de variation pour ledit groupe et les probabilités de variation pour les autres groupes, le second critère de performance d'identification étant atteint si ledit écart est supérieur à un quatrième seuil prédéfini. Notamment, les groupes microbiens sont des sérotypes ou des séquence types.

Selon un mode de réalisation, le procédé comporte en outre la recherche de causes racines lors d'une contamination d'un produit alimentaire.

Selon un mode de réalisation, la mesure du profil génétique du microorganisme est réalisé au moyen d'une amplification de séquences génétiques cibles sans mise en oeuvre de séquençage complet, en particulier une réaction de polymérisation en chaine.

L'invention a également pour objet un système de typage *in vitro* d'un microorganisme compris dans un échantillon, comprenant :

a. des moyens de stockages informatiques comprenant :

- une base de données de profils génétiques de souches microbiennes appartenant à l'espèce microbienne dudit microorganisme, lesdits profils consistant en la présence ou l'absence d'un ensemble prédéfini de marqueurs génétiques ;
- un arbre phylogénétique de l'espèce dudit microorganisme, des assignations des profils génétiques de la base de données à des positions dans l'arbre phylogénétique, de fréquences de variation des profils génétiques des souches microbiennes de la base de données pour des niveaux prédéfinis de l'arbre phylogénique ;

b. des moyens pour collecter et mémoriser une mesure du profil génétique du microorganisme ;
c. des moyens mis en oeuvre par ordinateur, de détermination pour au moins un niveau de l'ensemble de niveaux prédéfinis de l'arbre phylogénétique :

- d'une probabilité de variation de chaque profil génétique de la base de données appartenant audit niveau par rapport au profil génétique mesuré, ladite probabilité de variation étant calculée en fonction des fréquences de variation audit niveau ;
- de l'appartenance du microorganisme à une subdivision dudit niveau si un premier critère de performance d'identification est atteint pour ladite subdivision, ledit premier critère étant atteint si au moins une probabilité de variation pour ladite subdivision est supérieure à un premier seuil prédéfini.

Un tel système est configuré pour mettre en oeuvre un procédé du type précité.

L'invention a également pour objet un produit programme d'ordinateur enregistré sur un support informatique lisible par ordinateur comprenant des instructions pour l'exécution de l'étape c. d'un procédé du type précité.

L'invention a également pour objet un produit programme d'ordinateur enregistré sur un support informatique lisible par ordinateur comprenant des instructions pour la construction d'un arbre phylogénétique en fonction d'un core-génome et des fréquences de variation d'un ensemble de marqueurs d'un génome accessoire à des niveaux de l'arbre phylogénétique.

L'invention a également pour objet une composition pour le typage *in vitro* de microorganismes compris dans un échantillon par amplification de séquences de nucléotides cibles, en particulier par une réaction de polymérisation en chaine, ladite composition comprenant un ensemble d'oligonucléotides pour l'amplification d'au moins l'ensemble de marqueurs génétiques obtenus conformément au procédé précité.

L'invention a également pour objet un procédé de préparation d'un échantillon biologique comprenant, ou susceptible de comprendre une souche bactérienne, en vue d'un typage de ladite souche par une réaction de polymérisation en chaine, comprenant la mise en contact dudit échantillon avec une composition précité. En particulier, le procédé comprend une lyse de l'échantillon issu de ladite mise en contact de manière à libérer du matériel génétique des bactéries de la souche bactérienne.

**BREVE DESCRIPTION DES FIGURES**

L'invention sera mieux comprise à la lecture de la description qui suit, donnée uniquement à titre d'exemple, et réalisée en relation avec les dessins annexés, dans lesquels :

- la **figure 1** est une vue d'ensemble d'un procédé selon l'invention;
- la **figure 2** est un organigramme détaillant une phase d'apprentissage d'un typage phylogénétique selon l'invention;
- la **figure 3** est un tracé illustrant une distance génomique décroissante pour la construction d'un arbre phylogénétique;
- la **figure 4** est une vue schématique d'un arbre phylogénétique
- la **figure 5** est un schéma illustrant le calcul des fréquences de variation d'un profil génétique selon l'invention pour un taxon de l'arbre phylogénétique;
- la **figure 6** est un organigramme illustrant la production d'un kit PCR utiliser pour le typage selon l'invention;
- la **figure 7** est un organigramme détaillant une phase de prédiction d'un type phylogénétique selon l'invention;
- la **figure 8** est un organigramme illustrant le calcul de seuils utilisés pendant la phase de prédiction;
- la **figure 9** est un organigramme illustrant une phase d'apprentissage pour un sérotypage selon l'invention;
- la **figure 10** est un organigramme illustrant une phase de prédiction de sérotype selon l'invention;
- la **figure 11** est un organigramme illustrant le calcul de seuils utilisés pendant la phase de prédiction de sérotype;
- la **figure 12** est un tracé de la décroissance de l'entropie de Shannon lors de la sélection de marqueurs génétiques constitutif d'un profil génétique pour le typage de Listeria;
- la **figure 13** est un ensemble de tracés de fréquences de variation des profils génétiques dans l'arbre phylogénétique de Listeria;
- la **figure 14** est un tracé du taux d'assignation sérotypique correcte de Listeria en fonction de la précision balancée;
- la **figure 15** est un ensemble de tracés de taux d'assignation phylogénétique correcte de Listeria en fonction d'une précision moyenne;
- la **figure 16** est un tracé de la décroissance de l'entropie de Shannon lors de la sélection de marqueurs génétiques constitutif d'un profil génétique pour le typage de Salmonelle;
- la **figure 17** est un ensemble de tracés de fréquences de variation des profils génétiques dans l'arbre phylogénétique de Salmonelle;
- la **figure 18** est un tracé du taux d'assignation sérotypique correcte de Salmonelle en fonction de la précision balancée;
- la **figure 19** est un ensemble de tracés de taux d'assignation phylogénétique correcte de Salmonelle en fonction d'une précision moyenne; et
- les figures **20 à 22** illustrent des architectures informatiques pour la mise en oeuvre du typage selon l'invention.

## DESCRIPTION DETAILLEE DE L'INVENTION

A. <u>Définitions</u>

Dans le présent document, les définitions suivantes s'appliquent :

- *Échantillon biologique :* désigne tout échantillon contenant ou suspecté de contenir un ou plusieurs microorganismes;
- *Génome de base* (ou "core genome" ou "génome coeur", noté "Gb") d'une espèce microbienne : désigne des parties du génome (gènes ou groupes de gènes) partagées par l'ensemble des souches microbiennes appartenant à la ou les espèces microbiennes considérées. Au sens de l'invention, le génome de base peut évoluer si l'ensemble de souches à partir duquel il est produit évolue. De préférence, le génome de base est construit à partir d'au moins 100 souches microbiennes différentes, et de préférence à partir d'au moins 1000 souches. On notera que les portions génomiques de souches microbiennes appartenant au génome de base peuvent différer l'une de l'autres. Par exemple, un gène particulier peut appartenir au génome de base et présenter plusieurs allèles différents. Dans ce qui suit, l'expression "génome de base" lorsqu'elle s'applique à une souche microbienne correspond à la portion du génome de la souche appartenant au génome de base de l'espèce.
- *Génome accessoire* d'une espèce microbienne (noté "Ga") : désigne des parties du génome (gènes ou groupes de gènes) d'un ensemble de souches bactériennes qui n'appartiennent pas au génome de base dudit ensemble. Ces parties du génome sont donc absentes du génome d'au moins une des souches bactériennes de l'ensemble des souches bactériennes considérées. Au sens de l'invention, le génome accessoire peut évoluer si l'ensemble de souches à partir duquel il est produit évolue. De préférence, le génome accessoire est construit à partir d'au moins 100 souches microbiennes différentes, et de préférence à partir d'au moins 1000 souches.
- *Marqueur génétique* : désigne un gène ou une séquence nucléotidique à une position déterminée du génome (également nommée "locus"), une séquence nucléotidique pouvant se résumer à un unique nucléotide comme par exemple deux allèles différant d'un seul nucléotide, ou à un polymorphisme d'un seul nucléotide dans le cadre d'une mutation (ou SNP pour "single nucléotide polymorphism") ;

- *Microorganisme* : désigne tous les organismes microscopiques incluant notamment les bactéries, les levures et les champignons ;
- *Arbre phylogénétique* d'une espèce microbienne : désigne un arbre représentant des relations d'évolution parmi un ensemble de souches microbiennes, relations déterminées en fonction de leur similarités et différences dans leurs génomes coeurs. Au sens de l'invention, un arbre phylogénétique peut s'enrichir si l'ensemble de souches à partir duquel il est produit évolue. De préférence, un arbre phylogénétique est construit à partir d'au moins 100 souches microbiennes différentes, et de préférence à partir d'au moins 1000 souches. Par convention, la racine d'un arbre phylogénétique est désignée comme le premier niveau de l'arbre et les feuilles de l'arbre comme le dernier niveaux. Un niveau "supérieur" à un autre correspond ainsi à un niveau plus proche de la racine.

B. Mode de réalisation de l'invention

Il est à présent décrit un typage probabiliste selon l'invention appliqué au suivi de contamination par des souches bactériennes d'une espèce au sein d'une organisation, en particulier dans les locaux de production d'aliments destinés à la consommation humaine, ainsi que la détection de leurs sérotypes.

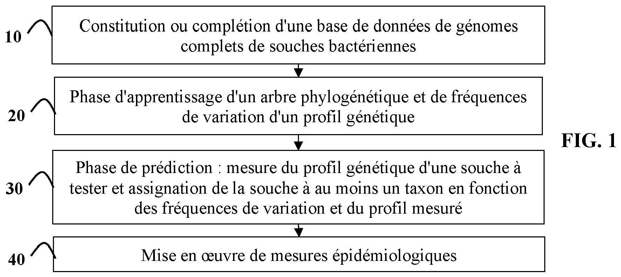

En se référant à la **figure 1,** le typage selon l'invention comprend les phases principales suivantes :

- une phase **10** de constitution d'une base de données de génomes complets de souches appartenant à l'espèce bactérienne;
- une phase **20** dite "d'apprentissage" ou "d'entrainement", visant à générer un arbre phylogénétique en fonction des génomes de la base de données, à choisir un profil génétique approprié et des fréquences de variation dudit profil génétique le long de l'arbre,
- une phase **30** dite "de prédiction" réalisant le typage d'une souche bactérienne inconnue en fonction de l'arbre et des fréquences produits lors de la phase **20**; et
- phases éventuellement complétées d'une phase **40** dite "épidémiologique" au cours de laquelle sont mises en oeuvre des mesures correctrices et/ou prophylactiques au sein de l'organisation en fonction du résultat du typage de la souche inconnue.

De préférence, la base de données des génomes complets est la plus représentative possible de la diversité génétique de l'espèce. Elle est avantageusement complétée au fil de l'eau en ajoutant des génomes complets de manière à viser cette diversité si sa version initiale est incomplète. Dans ce cas, le typage selon l'invention est mis en oeuvre lorsque ladite base comprend au moins 100 génomes de l'espèce, de préférence au moins 1000 génomes et de manière encore plus privilégiée plusieurs milliers de génomes.

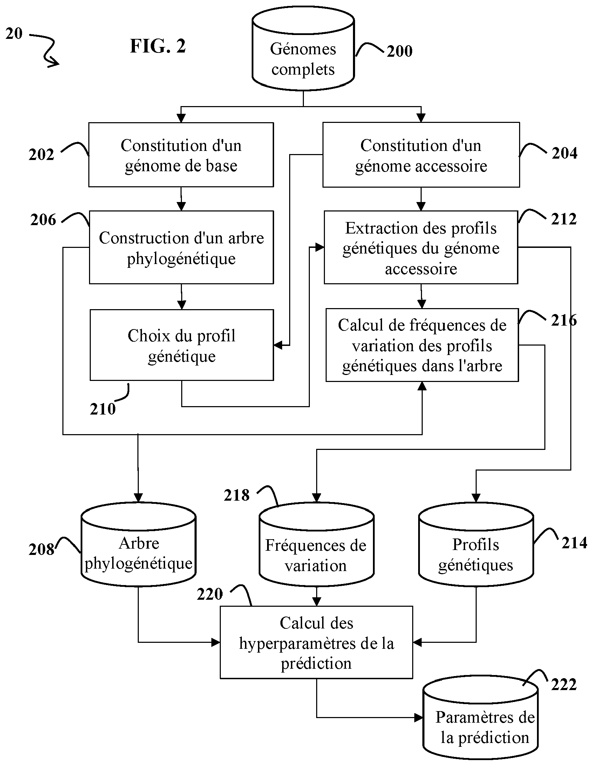

En se référant aux **figures 2, 3** et **4,** la phase d'entrainement **20** débute par la production d'un génome de base en **202** et d'un génome accessoire en **204** à partir des génomes de la base de données **200.** Dans une première variante, les génomes comprennent des annotations catégorisant les gènes comme appartenant à l'un ou l'autre de ces génomes. Dans une seconde variante, ces derniers sont produits de manière *ad hoc* depuis la base de données, les gènes présentant une fréquence allélique supérieure à un seuil, par exemple 95%, appartenant au génome de base, sinon au génome accessoire. Dans une troisième variante, les génomes de base et accessoire sont produits depuis la base de données par comparaison à un génome de référence de l'espèce, mémorisé dans la base, les gènes présentant une fréquence allélique comparativement au génome de référence supérieure à un seuil, par exemple 95%, appartenant au génome de base, sinon au génome accessoire. Dans une quatrième variante, la base de données est déjà constituée pour l'espèce ainsi qu'une version de son génome de base et accessoire.

Le typage selon l'invention se poursuit, en **206,** par la génération d'un arbre phylogénétique en fonction du génome de base. Plusieurs techniques pour construire un tel arbre sont possibles, telles que décrites par exemple dans les documents "Phylogenetics Algorithms and Applications" (Hu et al., Ambient Communications and Computer Systems, 2019), "A Review: Phylogeny Construction Methods " (Shaktawat et al., International Journal of Emerging Science and Engineering, 2019) ou "Essential Bioinformatics" (chapitre 11 de Jin Xiong, Cambridge University, 2011).

Dans une variante de l'invention, l'arbre est construit à partir d'un codage cg-MLST (pour "core-genome multi locus sequencing typing") des génomes de base. Ces derniers sont traduits en profils cg-MLST en utilisant par exemple le logiciel BioNumerics (bioMérieux, Marcy l'Etoile, France), SeqSphere+ (Ridom, Münster, Germany), BIGSdb (accessible à https://github.com/kjolley/BIGSdb et décrit dans le document de Schürch, A. et al. "Whole genome sequencing options for bacterial strain typing and épidémiologie analysis based on single nucléotide polymorphism versus gene-by-gene-based approaches". Clin. Microbiol. Infect., 2018), ou chewBBACA (accessible à https://github.com/B-UMMI/chewBBACA et décrit dans le document de Silva M et al., "chewBBACA: a complété suite for gene-by-gene schéma création and strain identification." Microb. Genom., 2018). Les profils cg-MLST ont pour avantage d'être des séquences de longueurs fixes, de quelques centaines de composants, et alignées les unes par rapport aux autres, permettant l'emploi direct de méthode de construction de l'arbre.

Cet arbre est avantageusement généré en appliquant une succession de regroupements par méthode de liens simples ("Single-linkage clustering" en anglais), et donc se fondant sur des distances génétiques entre les génomes de base, par exemple calculées à l'aide de la distance de Hamming normalisée. La subdivision d'un taxon de l'arbre consiste alors à regrouper dans un même sous-groupe les génomes de base différant l'un de l'autre par une distance inférieure à un seuil prédéfini. A titre d'exemple, des logiciels comme HierCC (accessible à https://github.com/zheminzhou/pHierCC et décrit dans le document "HierCC: a multi-level clustering scheme for population assignments based on core genome MLST" de Zhou et al., Bioinformatics, 2021) et Pathogenwatch du Center for Genomic Pathogen Surveillance (accessible à https://pathogen.watch/) peuvent être utilisés. Une fois l'arbre construit, ce dernier est mémorisé dans une base de données **208.**

Dans une variante particulièrement privilégiée de l'invention, le nombre de niveaux de l'arbre et les distances de clustering sont choisis de manière à produire au moins un niveau dans l'arbre tel que la diversité du génome de base à ce niveau correspond ou est proche de la diversité clonale observée dans l'espèce, de sorte que les taxons à ce niveau permettent de distinguer différents complexes clonaux parmi les souches bactériennes correspondant aux génomes complets de la base de données. Par exemple pour les *Salmonelles* et les *Listéria*, le niveau présente entre 150 et 350 différences dans le génome de base des souches, de préférence entre 200 et 300 différences. En particulier, la distance de clustering est décroissante depuis la racine vers les feuilles, par exemple linéairement.

La phase d'apprentissage **20** se poursuit par le choix, en **210,** d'un type de profils génétique dans le génome accessoire, à savoir un ensemble de marqueurs génétiques du génome accessoire de l'espèce dont l'absence et la présence sont mesurées dans une souche à tester lors de la phase de prédiction **30.** Le choix du profil consiste à choisir un niveau dans l'arbre, et de manière privilégiée un des niveaux représentant la diversité clonale, puis à choisir un ensemble de marqueurs ayant une entropie ou un degré d'impureté minimale à ce niveau. Le nombre total $N_M$ de marqueurs sélectionné pour construire le profil génétique est avantageusement sélectionné en fonction de la plateforme PCR particulière qui est utilisé pour réaliser le typage. Par exemple, pour la plateforme GENE-UP de la demanderesse, ce nombre $N_M$ est égal à 23 marqueurs pour les Salmonelles et 16 marqueurs pour les Listeria monocytogènes.

En pratique, l'ensemble de marqueurs est choisi de manière à minimiser un critère d'entropie (par exemple l'entropie de Rényi, de Hartley, de Shannon, de collision, ou l'entropie min) ou un critère d'impureté de Gini, en mettant en oeuvre un algorithme glouton ("greedy algorithm" en anglais).

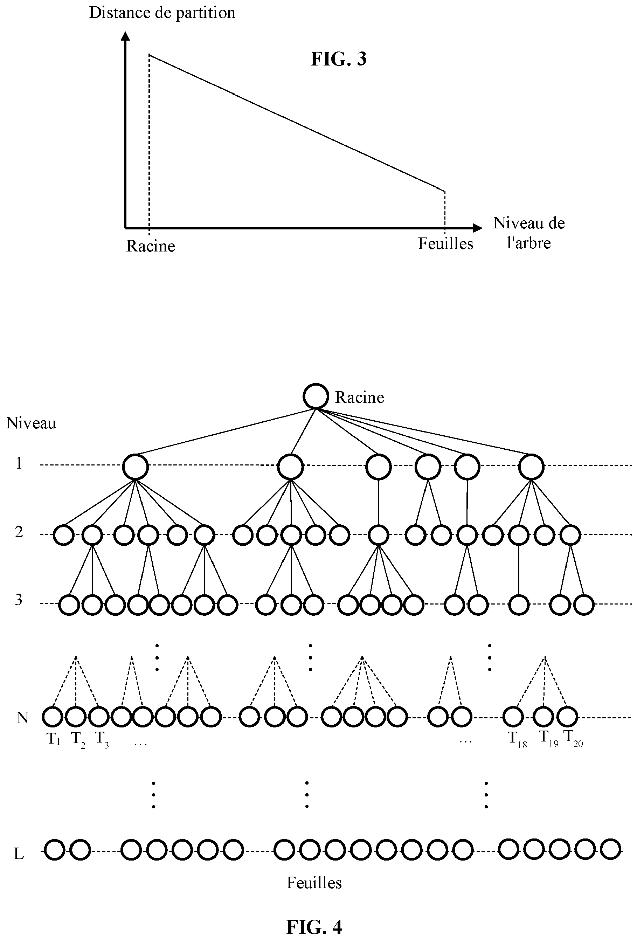

A titre d'illustration, et en se référant à l'arbre phylogénétique de la **figure 4,** la sélection d'un ensemble de marqueurs minimisant le critère d'entropie de Shannon du niveau N constitué de $Q_N = 20$ taxons $T_1$, $T_2$,..., $T_{20}$ consiste à choisir un premier marqueur $M_1$ du génome accessoire minimisant le critère d'entropie :

$$E_1 = -\sum_{i=1}^{Q_N} p(i) \log_2 p(i)$$

où $p(i) = \frac{n_i}{NG}$ avec *NG* le nombre total de génomes de base utilisés pour construire l'arbre phylogénétique et $n_i$ le nombre de fois que le marqueur est présent dans les génomes accessoires dans le i$^{\text{ème}}$ taxon $T_i$. Une fois le marqueur $M_1$ sélectionné, cette procédure est relancée pour sélectionner le second marqueur $M_2$ dans le génome accessoire retranché du marqueur $M_1$. Ainsi le j$^{\text{ème}}$ marqueur $M_j$ est le marqueur tel que

$$M_j = arg \min_{M_j \in Ga, M_j \notin \{M_1, M_2, ..., M_{j-1}\}} -\sum_{i=1}^{Q_N} p(i) \log_2 p(i)$$

$p(i) = \frac{n_i}{NG}$ avec *NG* le nombre total de génomes de base utilisés pour construire l'arbre phylogénétique et $n_i$ est le nombre de fois que le marqueur $M_j$ est présent dans les génomes accessoires dans le i$^{\text{ème}}$ taxon $T_i$.

En variante, la sélection pas à pas des marqueurs est réalisée en minisant l'entropie conditionnelle du niveau N selon la formule:

$$M_j = arg \min_{M_j \in Ga, M_j \notin \{M_1, M_2, ..., M_{j-1}\}} - \sum_{i=1}^{Q_N} \sum_{l=1}^{2^j} p(i)p(M_l|i) \log_2 p(i)p(M_l|i)$$

où :

- $p(i) = \frac{n_i}{NG}$ avec $NG$ le nombre total de génomes de base utilisés pour construire l'arbre phylogénétique et $n_i$ le nombre de fois que le marqueur est présent dans les génomes accessoires dans le i$^{ème}$ taxon $T_i$;

- et $p(M_l|i) = \frac{n_l}{N_i}$ avec $n_l$ le nombre de fois que la 1$^{ième}$ valeur de l'ensemble de marqueur $\{M_1, M_2, ... , M_{j-1}, M_j\}$, parmi les $2^j$ valeurs possibles dudit ensemble, est présente dans le ième taxon $T_i$ et $N_i$ le nombre de génomes de base compris dans le taxon $T_i$.

Les inventeurs ont observé que le typage selon l'invention nécessite au plus 50 marqueurs, de préférence moins de 30 marqueurs, plus particulièrement un nombre compris en 16 et 30 marqueurs, pour obtenir des performances de typage élevées.

La phase d'apprentissage **20** se poursuit, en **212,** par l'extraction des génomes accessoires des profils génétiques constitués des marqueurs sélectionnés, profils mémorisés dans une base de données **214.** Le profil génétique d'un génome accessoire est donc un vecteur $P$ de $\{0,1\}^{N_M}$, la j$^{ième}$ composante $P(j)$ du vecteur $P$ correspondant à la valeur du marqueur $M_j$, cette valeur étant égale à 0 lors le marqueur $M_j$ est absent et à 1 lorsqu'il est présent.

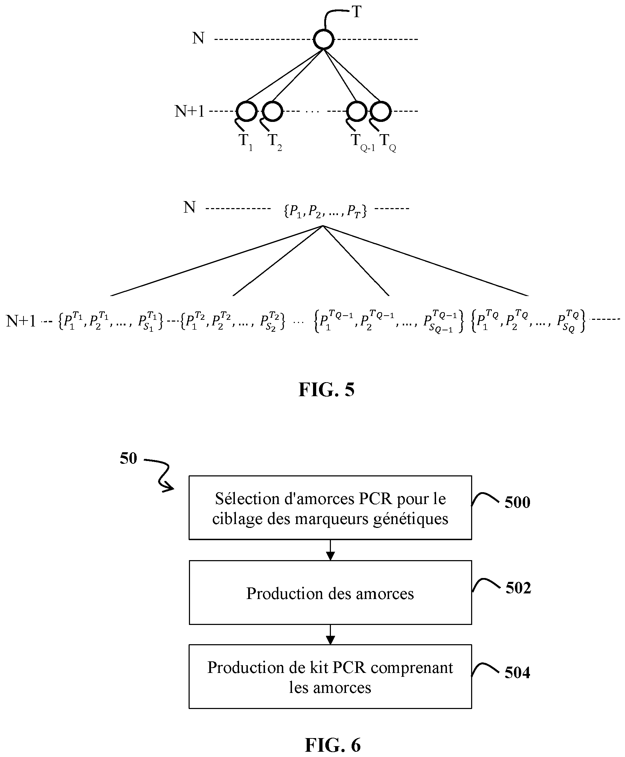

Une fois la base de profils **214** constituée, des fréquences de variation des profils le long de l'arbre sont calculées en **216.** En se référant à la **figure 5,** pour chaque taxon $T$ de l'arbre qui est partitionné en subdivisions $T_1, T_2, ..., T_Q$, il existe un ensemble de profils génétiques $\{P_1, P_2, ... , P_T\}$ associé au taxon $T$ partitionné en sous-ensembles de profils

$$\left\{P_1^{T_1}, P_2^{T_1}, ..., P_{S_1}^{T_1}\right\}, \quad \left\{P_1^{T_2}, P_2^{T_2}, ..., P_{S_2}^{T_2}\right\}, ..., \left\{P_1^{T_Q}, P_2^{T_Q}, ..., P_{S_Q}^{T_Q}\right\}$$ respectivement associés aux sous-taxons $T_1, T_2, ..., T_Q$. Pour chaque marqueur $M_j$ du profil génétique, il est calculé une fréquence de variation $P(M_j|T)$ de ce marqueur pour le taxon $T$ comme étant égal au nombre, divisé par $Q$, de sous-taxons $T_1, T_2, ..., T_Q$ pour lesquels le marqueur $M_j$ prend à la fois la valeur 0 et la valeur 1, à savoir une fréquence selon la relation :

$$P(M_j|T) = \frac{card\left(\left\{T_q \backslash \exists\ (v, w), u \neq w, P_u^{T_q}(j) = 0\ \&\ P_w^{T_q}(j) = 1\right\}\right)}{Q}$$

où $card(E)$ désigne la fonction cardinalité d'un ensemble $E$. Le calcul des fréquences produit ainsi pour chaque taxon $T$ subdivisé, un vecteur de fréquences $P(.|T)$ de $[0,1]^{N_M}$ tel que $P(.|T)(j) = P(M_j|T)$. Un fois calculés, les vecteurs de fréquence sont mémorisés dans une base de données **218.**

La phase d'apprentissage **20** se termine par le calcul, en **220,** d'une grille d'hyperparamètres utilisés lors de la phase de prédiction **30,** calcul qui sera détaillé ci-après une fois décrit la prédiction **30,** paramètres étant mémorisés dans une base de données **222.**

En résumé, la phase d'apprentissage **20** fournit un arbre phylogénétique dans lequel chaque génome complet et son profil génétique extrait de son génome accessoire est positionné dans l'arbre et à chaque taxon de l'arbre, à l'exclusion des feuilles, est associé une fréquence de variation de chaque marqueur génétique du profil.

En se référant à la **figure 6,** l'invention comporte la conception et la production **50** d'une composition, ou "kit", pour la mise en oeuvre d'une amplification visant la détection des marqueurs sélectionnée, comprenant :

- la sélection, en **500,** des amorces d'amplification pour l'amplification des marqueurs génétiques sélectionnés:
- la production desdites amorces en **502** et si nécessaire la production de sondes de détection de manière à détecter la présence d'un marqueur dans un moins un de ses variants, et de manière préférentielle dans le maximum de ses variants;
- la production, en **504,** d'une solution tampon comprenant les amorces sélectionnées, les sondes de détection si nécessaire, des dNTP (désoxyribonucléotide triphosphates) apportant l'énergie et les bases nucléotidiques nécessaires à la production d'amplicons, une enzyme d'amplification et des sels tels que les ions Mg$^{2+}$ ou NaCl permettant le bon fonctionnement de l'enzyme, à des concentrations appropriées. Des colorants peuvent être ajoutés à cette solution.

- la production d'un tampon de lyse comprenant des billes par exemple des billes magnétiques ou de céramique, éventuellement un colorant.

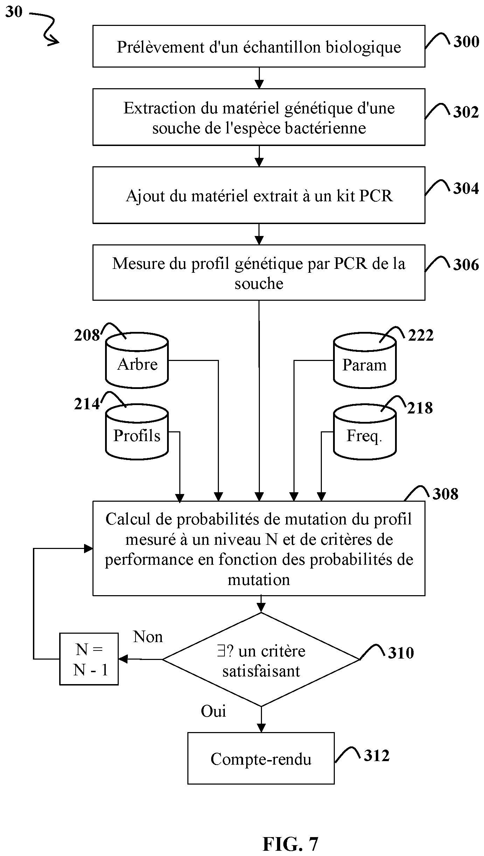

Les kits produits sont ensuite utilisés pendant la phase de prédiction **30**. En se référant à la **figure 7,** cette phase débute par le prélèvement **300** d'un échantillon biologique dans l'organisation, par exemple une usine de production de produit alimentaire. Lors d'une étape **302** suivante, l'échantillon est préparé de manière à détecter un pathogène donné par des méthodes connues de l'état de la technique telles qu'une culture sur boite, une détection immunologique de type VIDAS®, ou encore par biologie moléculaire par exemple avec la plateforme GENE-UP®. Suite à la détection dudit pathogène, une étape de confirmation peut avoir lieu.

Parallèlement, si la colonie est identifiée comme appartenant à l'espèce, la phase de prédiction **30** se poursuit en **302** à partir de colonies isolées ou d'un bouillon d'enrichissement en vue de sa caractérisation par une amplification PCR. En particulier, l'échantillon subit un ensemble d'étapes préalables, telle qu'une lyse pour libérer l'ADN des bactéries la constituant, optionnellement une extraction de l'ADN issu de la lyse au moyen de billes, par exemple des billes magnétiques ou céramiques. Une fois l'ADN de la souche bactérienne libéré, il est ajouté, en **304,** au kit PCR conçu pour cibler *a minima* les marqueurs génétiques sélectionnés lors de l'étape d'apprentissage **20.** Enfin, la PCR est mise en oeuvre en **306** afin de mesurer le profil génétique de la souche, par exemple au moyen de la plateforme GENE-UP® de la Demanderesse.

Le profil mesuré, noté $P_{mes}$, est ensuite analysé, en **308,** afin de positionner la souche bactérienne dans l'arbre phylogénétique. Partant d'un niveau $N$ de l'arbre, par exemple le niveau directement supérieur au niveau des feuilles, l'analyse **308** comprend les étapes suivantes :

a. pour chaque taxon du niveau $N,$ noté $T_{i,N}$ (ième taxon du Nième niveau):

a1. pour chaque profil mémorisé, noté $P_{obs}$, de la base de données **214** qui est assigné au taxon $T_{i,N}$, le calcul d'une probabilité $P_{mut}(P_{mes}, P_{obs})$ que le profil mesuré $P_{mes}$ soit une variation du profil mémorisé $P_{obs}$ selon la relation:

$$P_{mut}(P_{mes}, P_{obs}) = \prod_{\substack{j=1,\dots,N_m \\ P_{mes}(j) \neq P_{obs}(j)}} P_{mut}\big(M_j \big| T_{i,N}\big)$$

On notera ainsi que si le profil mesuré $P_{mes}$ est identique au profil $P_{obs}$ alors cette probabilité est égale à 1 (aucune variation) et diminue rapidement si le profil $P_{mes}$ s'écarte du profil $P_{obs}$, et ce d'autant plus que les fréquences $P_{mut}$ $(M_j|T_{i,N})$ sont faibles.

a2. sélection du maximum $P_{mut}^{max}(i, N)$ des probabilités $P_{mut}(P_{mes}, P_{obs})$ calculées pour le taxon $T_{i,N}$.

b. Assignation, en **310,** du profil mesuré $P_{mes}$ à un des taxons $T_{j,N}$ du niveau N tel que :

$$\begin{cases} T_{j,N} = arg \max_i P_{mut}^{max}(i, N) \\ P_{mut}^{max}(j, N) \geq S_1 \\ \forall i \neq j, P_{mut}^{max}(j, N) - P_{mut}^{max}(i, N) \geq S_2 \end{cases}$$

où $S_1$ et $S_2$ sont des seuils positifs non nuls prédéterminés.

c. Si aucun taxon du niveau N vérifie le critère de l'étape b, passage au niveau supérieur N-1 de l'arbre et mise en oeuvre des étapes a et b pour ce nouveau niveau.

Une fois le profil $P_{mes}$ assigné à un niveau de l'arbre phylogénétique et un taxon de ce niveau, la phase de prédiction **30** délivre un compte rendu à l'utilisateur, par exemple sous forme d'affichage sur un écran et/ou de fichiers mémorisés sur un ordinateur et/ou de message (*e.g.* email) à l'attention de l'utilisateur. Si deux taxons ont des résultats identiques à l'issue de l'étape b, ces deux taxons sont inclus ou bien il est noté un typage non conclusif dans le compte rendu.

La position de la nouvelle souche bactérienne testé dans l'arbre phylogénétique permet de typer cette dernière par rapport aux souches bactériennes correspondant aux génomes complets de la base de données **200.** Outre ce type en référence à des souches préalablement observées et capitalisées dans les différentes bases de données, le typage selon l'invention permet le typage relatif de deux souches bactériennes si ces deux souches sont assignées au même taxon. De plus, l'invention permet de définir la lignée d'une souche par rapport à celles des bases de données ou par rapport à une autre souche testée. En effet, la position relative de deux souches dans l'arbre permet de caractériser leur évolution l'une par rapport à l'autre.

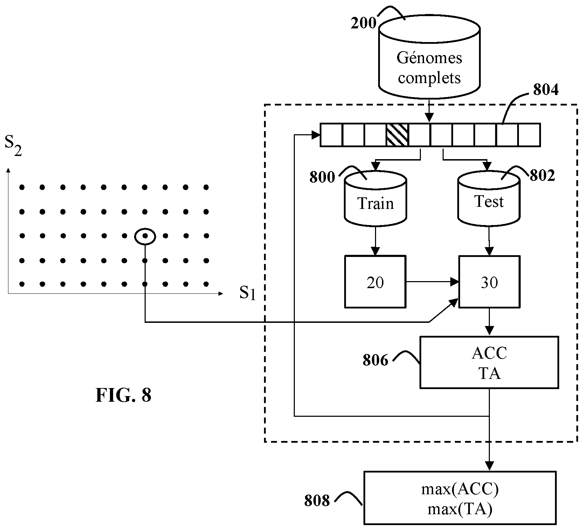

Les valeurs des seuils $S_1$ et $S_2$ sont des hyperparamètres déterminés lors de l'étape **220** de la phase d'apprentissage **30,** de préférence au moyen d'une validation croisée. En se référant à la **figure 8,** une grille de valeurs pour les seuils $S_1$ et $S_2$ est tout d'abord déterminée, et pour chaque couple de seuils de la grille, une détermination du taux correct d'assignation et la précision moyenne est mise en oeuvre par :

- la partition de la base de génome complets **200** en une base d'entrainement **800** et une base de test **802** selon une stratégie de validation croisée **804,** par exemple une stratégie dite des 10 plis ("ten-folds cross validation" en anglais);

- la mise en oeuvre de la phase d'apprentissage **20** sur la base d'entrainement **802;**

- la mise en oeuvre de la phase de prédiction **30** pour chaque profil génétique issu de la base de test **802** et le calcul en **806** de la précision moyenne ACC et le taux d'assignation correct TA des profils génétiques de la base **802** dans l'arbre phylogénétique;

- la sélection, en **808,** de la valeur maximale des précisions moyennes et des taux d'assignation correcte parmi les plis de la stratégie de la validation croisée. Ces deux valeurs correspondent donc respectivement à la précision moyenne et au taux d'assignation correcte de la phase de prédiction phylogénétique **30** pour les seuils $S_1$ et $S_2$. Le couples de seuils présentant les meilleurs précision et taux sont retenus pour le typage phylogénétique selon l'invention. En variante, l'utilisateur peut choisir la précision et/ou le taux d'assignation qu'il désire et le typage phylogénétique sélectionne le couple de seuils dont la précision et le taux est le plus proche de ceux choisis par l'utilisateur.

De manière privilégiée, le nombre de niveaux dans l'arbre phylogénétique et la loi de décroissance des distances de clustering, par exemple affine, sont également des hyperparamètres, le procédé décrit en relation avec la **figure 8** étant donc complété par la sélection d'une grille pour ces deux paramètres, et le parcours de ladite grille conjointement avec le parcours de la grille de seuils $S_1$ et $S_2$.

Il est à présent décrit une variante privilégiée de l'invention consistant à prédire l'appartenance d'une souche bactérienne à un groupe particulier de l'espèce bactérienne en fonction de son profil génétique mesuré. Dans l'exemple illustré, ces groupes sont des sérotypes mais cette variante s'applique à toute subdivision explicite à un niveau de l'espèce bactérienne. On notera que cette subdivision de l'espèce ne correspond pas au premier niveau de l'arbre phylogénétique et est définie indépendamment de ce dernier.

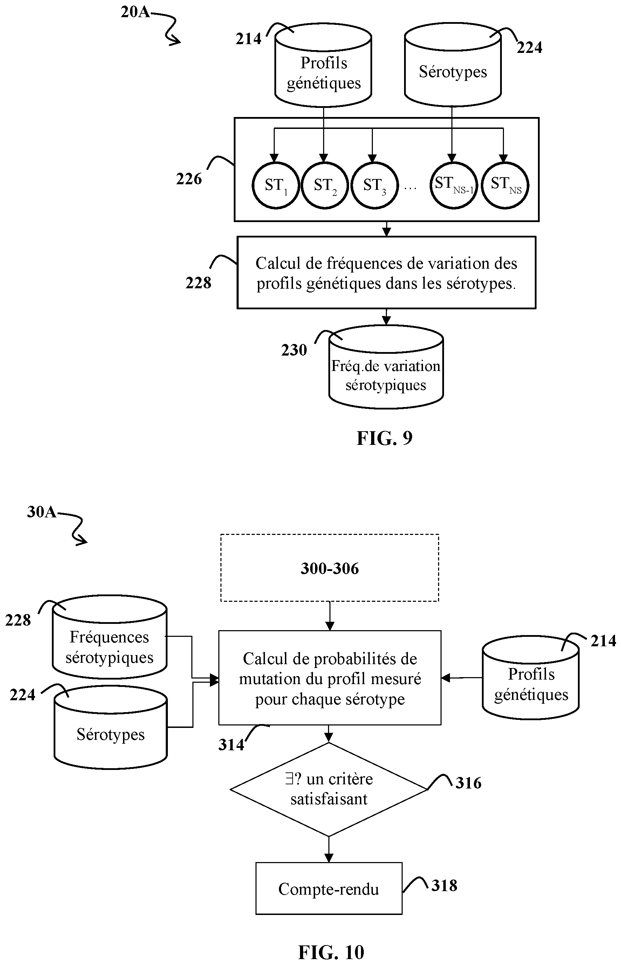

En se référant à la **figure 9,** la phase d'apprentissage **20** décrite ci-avant en relation avec la **figure 2** est complétée par un apprentissage sérotypique **20A** comprenant :

a. la collecte des sérotypes de toute ou partie des souches bactériennes correspondant aux génomes complets, sérotypes mémorisés dans une base de données **224;**

b. la répartition, en **226,** des profils génétiques observés de la base de données **214** des souches sérotypées parmi les $NS$ différents sérotypes $ST1, ST2, ...ST_{NS}$. Les profils génétiques associés à un sérotype sont ci-après qualifiés de "sérotypés";

c. le calcul, **228,** des fréquences de variation des profils génétiques sérotypés. Notamment, pour chaque marqueur $M_j$, sa fréquence de variation $P(M_j|ST_i)$ dans un sérotype $STi$ est égal au nombre de fois où ce marqueur prend la valeur 1 sur le nombre total de profils génétiques assigné au sérotype $STi$;.

d. la mémorisation dans une base de données **230** des fréquences de variation $P(M_j|ST_i)$ des profils génétiques sérotypés dans les différents sérotypes.

Si les éléments de la phase d'apprentissage **20** décrits en relation avec la figure 2 restent inchangées, signifiant notamment que le type de profils génétiques utilisés est déterminé en fonction de l'arbre phylogénétique et non pas de la subdivision en sérotype, la phase de prédiction **30** peut prendre deux formes. Dans la première, la phase **30** déjà décrite est complétée par une prédiction du sérotype d'une nouvelle souche à tester, en parallèle des étapes **308-312** de la figure 2. Dans la seconde forme, seule ladite prédiction est uniquement mise en oeuvre et remplace les étapes **308-312.**

En se référant à la **figure 10,** la prédiction **30A** du sérotype d'une nouvelle souche bactérienne fait suite aux étapes **300-306** mesurant le profil génétique d'une souche bactérienne de l'espèce contenue dans un échantillon biologique, et comprend les étapes suivantes :

a. pour chaque sérotypes, noté $ST_i$:

a1. pour chaque profil génétique sérotypé, noté $P_{ST}$, du groupe $ST_i$ de la base de données 228, le calcul d'une probabilité $P_{mut}(P_{mes}, P_{ST})$ que le profil mesuré $P_{mes}$ soit une variation du profil mémorisé $P_{ST}$ selon la relation:

$$P_{mut}(P_{mes}, P_{ST}) = \prod_{\substack{j=1,...,N_m \\ P_{mes}(j) \neq P_{ST}(j)}} P_{mut}(M_j | ST_i)$$

a2. sélection du maximum $P_{mut}^{max}(ST_i)$ des probabilités $P_{mut}(P_{mes}, P_{obs})$ calculées pour le serotype $ST_i$;

b. Assignation du profil mesuré $P_{mes}$ à un des sérotypes $ST_j$ tel que :

$$\begin{cases} ST_j = arg \max_i P_{mut}^{max}(ST_i) \\ P_{mut}^{max}(ST_j) \geq S_3 \\ \forall i \neq j, P_{mut}^{max}(ST_j) - P_{mut}^{max}(ST_i) \geq S_4 \end{cases}$$

où $S_3$ et $S_4$ sont des seuils positifs non nuls prédéterminés ;
c. Production et délivrance, en **318,** d'un compte rendu sur le sérotype détecté pour la souche bactérienne objet du typage sérotypique.

Dans une variante du sérotypage **30A,** si la seconde condition $P_{mut}^{max}(ST_j) - P_{mut}^{max}(ST_i) \geq S_4$ n'est pas satisfaite, le sérotypage renvoie les deux ou trois premiers sérotypes associés aux probabilités $P_{mut}^{max}(ST_i)$ les plus élevées, ainsi que lesdites probabilités.

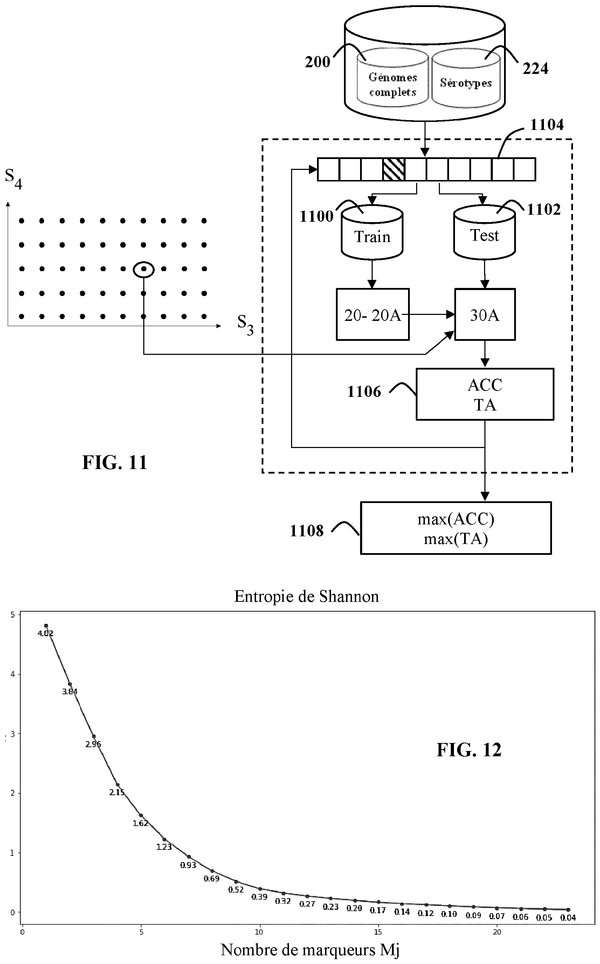

Les valeurs des seuils $S_3$ et $S_4$ sont des hyperparamètres déterminés lors de l'étape **220** de la phase d'apprentissage **30,** de préférence au moyen d'une validation croisée. En se référant à la **figure 11,** une grille de valeurs pour les seuils $S_3$ et $S_4$ est tout d'abord déterminée, et pour chaque couple de seuils de la grille, une détermination du taux correct d'assignation et la précision moyenne pour le typage sérotypique est mise en oeuvre par :

- la partition de la bases de génome complets **200** et des sérotypes **224** en une base d'entrainement **1100** et une base de test **1102** selon une stratégie de validation croisée **1104,** par exemple une stratégie dite des 10 plis ("ten-folds cross validation" en anglais);

- la mise en oeuvre de la phase d'apprentissage **20 - 201** sur la base d'entrainement **1102;**

- la mise en oeuvre de la phase de prédiction **30A** pour chaque profil génétique issu de la base de test **1102** et le calcul en **1106** de la précision moyenne ACC et le taux d'assignation correct TA des profils génétiques de la base **802** dans les sérotypes;

- la sélection, en **1108,** de la valeur maximale des précisions moyennes et des taux d'assignation correcte parmi les plis de la stratégie de la validation croisée. Ces deux valeurs correspondent donc respectivement à la précision moyenne et au taux d'assignation correcte de la phase de prédiction sérotypique **30A** pour les seuils $S_3$ et $S_4$. Le couples de seuils présentent les meilleurs précision et taux sont retenus pour le typage sérotypique selon l'invention. En variante, l'utilisateur peut choisir la précision et/ou le taux d'assignation qu'il désire et le sérotypage selon l'invention sélectionne le couple de seuils dont la précision et le taux est le plus proche de ceux choisis par l'utilisateur.

Dans la variante venant d'être décrite, la détermination du sérotype d'une souche à tester comprend les calculs tels que décrits en relation avec les figures 9 et 10. Dans une autre variante, pour chaque taxon de l'arbre phylogénétique, il est calculé la problabilité de chaque sérotype dans ce taxon en fonction de la base de données de sérotypes **224.** Par exemple, la probabilité d'un sérotype donné $ST_j$ dans le taxon $T_i$ est égale au nombre de génomes groupés dans ce taxon qui présentent ce sérotype divisé par le nombre total de génomes du taxon. Ces probabilités sont mémorisées dans une table de référence ou un dictionnaire et la prédiction du sérotype d'une souche à tester consiste d'abord à déterminer le taxon de l'arbre phylogénétique auquelle elle appartient de la manière décrite aux étapes **308-312.** Une fois le taxon identifié, le sérotypage de la souche consiste à interroger les probabilités de la table de référence associées audit taxon et à délivrer au moins le sérotype ayant la plus grande probabilité à l'utilisateur.

D. Exemples de réalisation

D. 1. *Listeria monocytogènes*

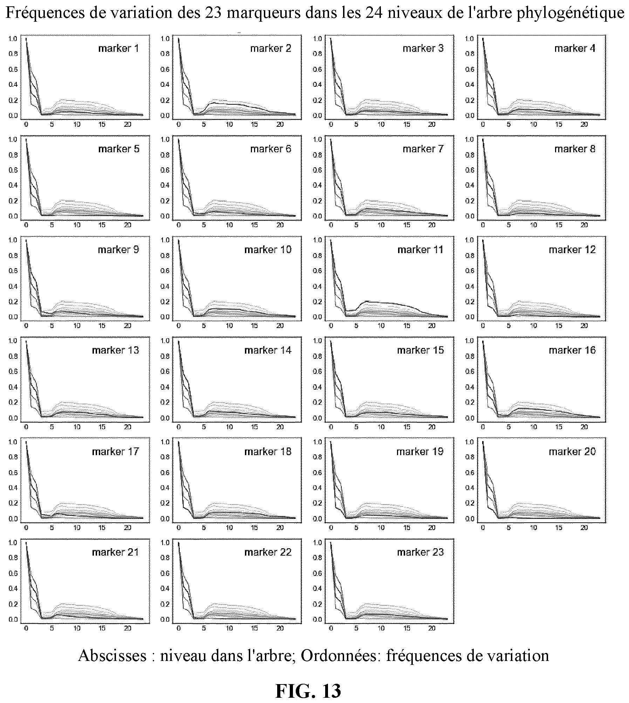

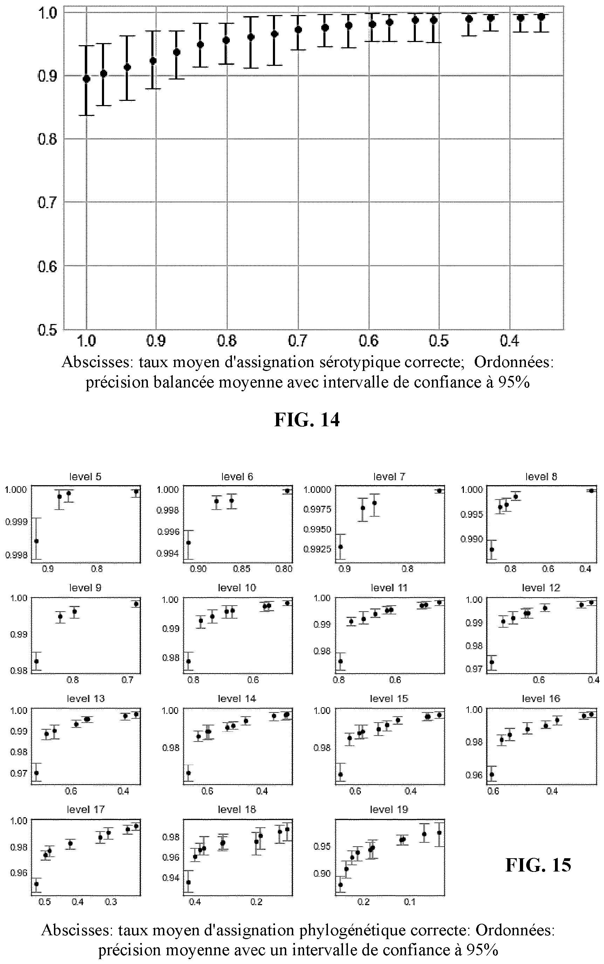

37000 souches de *Listeria* ont été collectées, complètement séquencées et sérotypées. Le nombre de niveaux dans l'arbre est égal à 23, à savoir le nombre total de cibles de la plateforme PCR GENE-UP® de la Demanderesse, et la loi de décroissance des distances est sélectionnée pour le clustering de manière à obtenir une diversité similaire à la diversité clonale à un niveau compris entre 10 et 15 dans un arbre phylogénétique comportant 24 niveaux. La **figure 12** illustre la décroissance de l'entropie de Shannon du profil génétique à mesure de la sélection des marqueurs dans le génome accessoire au niveau 13 de l'arbre phylogénétique, l'entropie du profil final de quelques pourcents seulement de l'entropie libre à ce niveau . La **figure 13** illustre les fréquences de variation du profil aux différents niveaux de l'arbre, une courbe dans un graphique correspondant à un taxon du niveau et la courbe en gras correspondant à la fréquence du marqueur étiqueté. Comme cela est visible sur la **figure 14,** qui illustre le typage sérotypique parmi 12 sérotypes connus des *Listeria*, pour un taux d'assignation sérotypique correct de 100%, la précision balancée moyenne est égale à 90%, démontrant l'efficacité de ce typage selon l'invention. Il est à noter qu'il est possible de choisir une précision supérieure pour un taux d'assignation inférieur satisfaisant. Notamment pour un taux de 80%, la précision est proche de 95% avec l'intervalle de confiance à 95% contenu entièrement dans le dernier décile. La **figure 15** illustre la précision moyenne d'un niveau en fonction du taux d'assignation phylogénétique correcte. Là encore, on note l'excellent niveau de performance de l'invention, ces deux valeurs étant respectivement supérieures à 95% et 70% pour tout niveau supérieur au niveau 15, et chacune proche de 100% pour les niveaux supérieurs au niveau 9.

D.2. *Salmonelle*

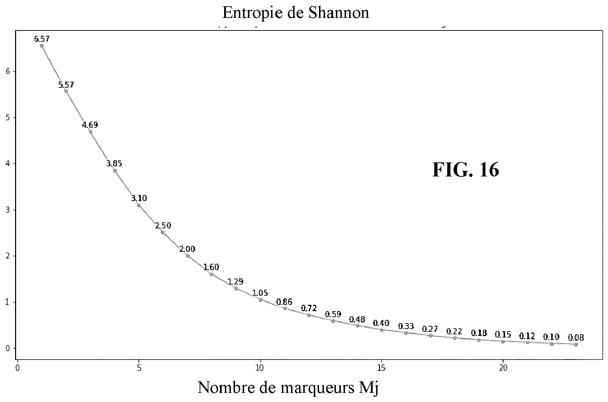

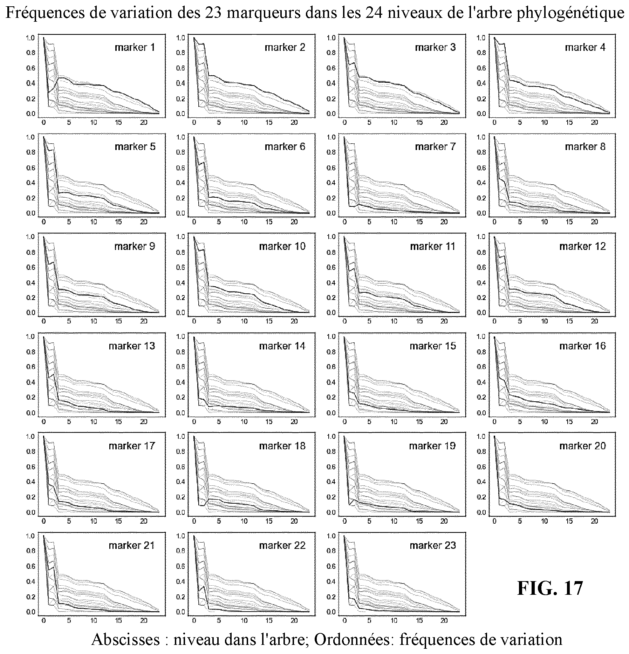

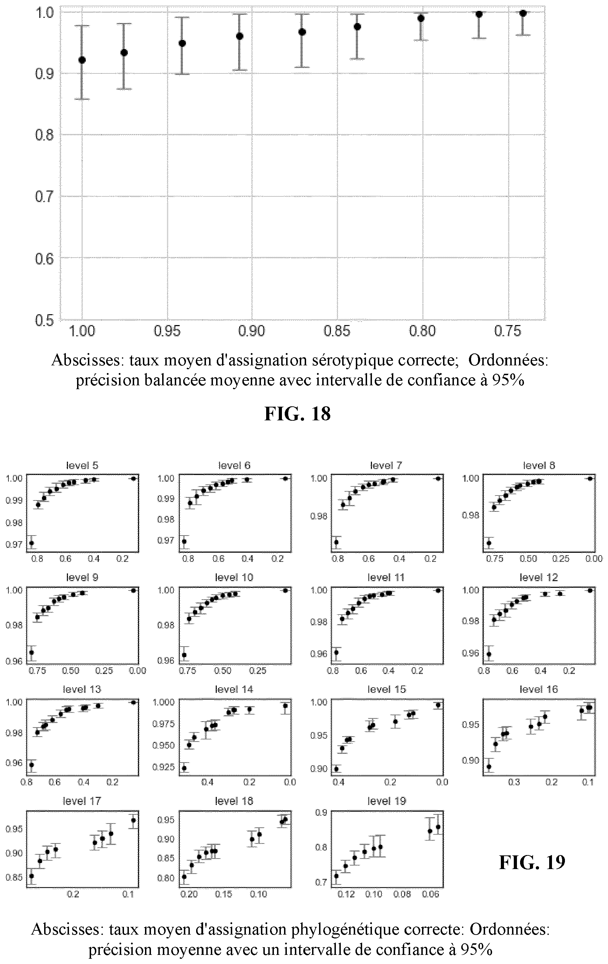

39000 souches de Salmonelle ont été collectées, complètement séquencées et sérotypées. Le nombre de niveaux dans l'arbre est égal à 23, à savoir le nombre total de cibles de la plateforme PCR GENE-UP® de la Demanderesse, et la loi de décroissance des distances est sélectionnée pour le clustering de manière à obtenir une diversité similaire à la diversité clonale à un niveau compris entre 10 et 15 dans un arbre phylogénétique comportant 24 niveaux. La **figure 16** illustre la décroissance de l'entropie de Shannon du profil génétique à mesure de la sélection des marqueurs dans le génome accessoire au niveau 13, l'entropie du profil final étant sensiblement nulle. La **figure 17** illustre les fréquences de variation du profil aux différents niveaux de l'arbre, une courbe dans un graphique correspondant à un taxon du niveau et la courbe en gras correspondant à la fréquence moyenne du niveau. Comme cela est visible sur la **figure 18,** qui illustre le typage sérotypique parmi les plus de 2.600 sérotypes connus de Salmonelles, pour un taux d'assignation sérotypique correct de 100%, la précision balancée moyenne est supérieure à 90%, démontrant l'efficacité de ce typage selon l'invention. Il est à noter qu'il est possible de choisir une précision supérieure pour un taux d'assignation inférieur satisfaisant. Notamment pour un taux de 80%, la précision est proche de 100% avec l'intervalle de confiance à 95% contenu entièrement dans le dernier décile. La **figure 19** illustre la précision moyenne d'un niveau en fonction du taux d'assignation phylogénétique correcte. Là encore, on note l'excellent niveau de performance de l'invention, ces deux valeurs étant respectivement supérieure à 95% et 80% pour tout niveau supérieur au niveau 13.

E. Mesures préventives et curative

La capacité de la solution à identifier facilement et rapidement des souches permet d'envisager son utilisation pour la recherche de causes racines lors d'une contamination d'un produit fini alimentaire identifié lors de contrôle de routines. Cela inclut notamment la recherche de la souche dans l'environnement de l'usine concernée, grâce à des prélèvement de surface à l'aide par exemple d'écouvillons, d'éponges ou de chiffonnettes. La recherche de la souche peut également se faire dans les matières premières impliquées dans la conception du produit fini concerné, afin de relier la contamination à un ou plusieurs fournisseurs et/ou transporteur(s) et/ou lieu(x) de stockage. Il peut également permettre d'identifier une contamination lors de la phase de détection en laboratoire, soit par une souche de laboratoire, soit par leur ADN, qui aurait biaisé le test libératoire ayant identifié la présence de l'espèce concernée. Dans ce cas, il permettrait d'établir un plan de retest du produit fini et d'éviter sa destruction. Enfin, la solution peut être utilisée lors d'une crise sanitaire afin de caractériser rapidement la/les souche(s) responsable(s) et de les relier à des souches identifiées dans des produits alimentaires, cosmétiques, vétérinaires, ou autres, incriminés. Son utilité couvre donc des besoins internes à une usine de production, par exemple de produits alimentaires, des besoins d'investigations lors de crises sanitaires ou lors d'audits réglementaires sur site. L'utilisation de cette solution permet ainsi de rationaliser et de mettre en place des mesures préventives et/ou correctives adaptées pour réduire voir supprimer les risques de récurrence des contaminations, et par conséquent des infections.

F. Implémentation informatique matérielle de l'invention

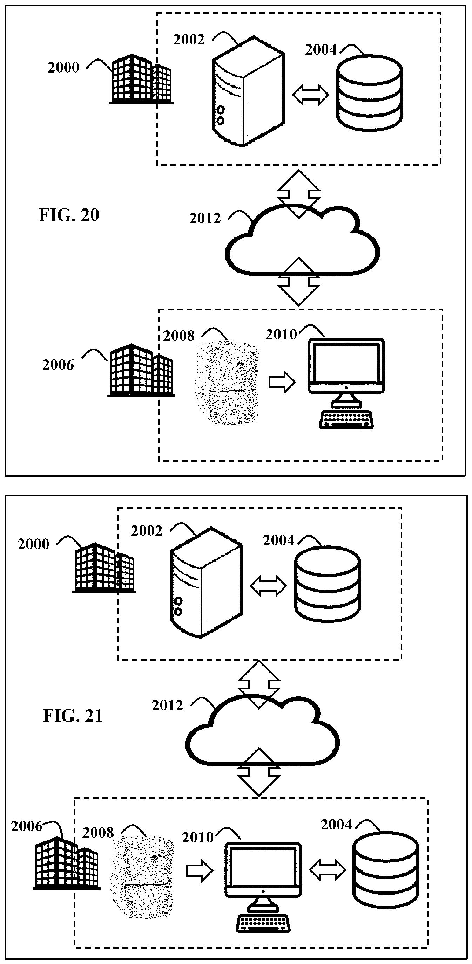

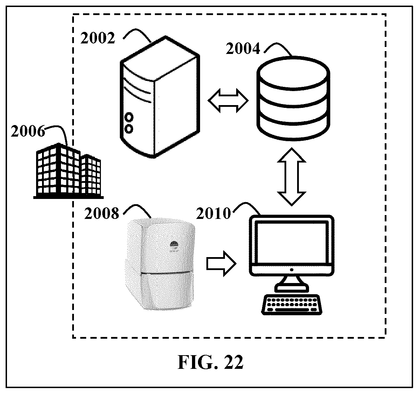

La phase d'apprentissage **20, 20A** et la phase de prédiction **30-30A,** hormis les étapes de préparation d'échantillon et de mesure du profil génétique des souches à tester sont mises en oeuvre par ordinateur, à savoir au moyen de circuits matériels comprenant des mémoires informatiques (cache, RAM, ROM...) et un ou plusieurs microprocesseurs ou processeurs, organisés ou non sous forme de noeuds de calcul, nécessaires à l'exécution d'instructions informatiques mémorisées dans les mémoires pour la mise en oeuvre desdites phases. Plusieurs architectures sont possibles, comme par exemple illustrées aux **figures 20-22.** Dans une première variante d'architecture (fig. 20), une première organisation **2000,** par exemple la Demanderesse, héberge ou contrôle un ou plusieurs serveurs de calcul **2002** associés à une ou plusieurs bases de données **2002** pour la mémorisation des génomes, de l'arbre phylogénétique, des fréquences phylogénétiques, des profils génétiques observées, des sérotypes et fréquences sérotypiques et la phase d'apprentissage **20, 20A** est mise en oeuvre par l'organisation 2000 sur son ou ses serveurs **2002.** Une seconde organisation **2006,** par exemple un laboratoire microbiologique, héberge une plateforme PCR **2008** connectée, ou incorporant, un ordinateur **2010** et met en oeuvre une partie de la phase de prédiction **30,** à savoir l'ensemble des étapes jusqu'à la mesure du profil génétique d'une souche bactérienne à tester. Le profil mesuré est alors poussé, au travers d'un réseau de connexion à distance, à la première organisation **2000** pour la mise en oeuvre sur le serveur **2002** du reste de la phase de prédiction **30, 30A.** Le compte rendu produit est alors poussé, au travers du réseau **2012,** vers la seconde organisation **2006** qui prend ou non des mesures épidémiologiques en fonction du compte rendu. Une seconde variante d'architecture (fig. 21) diffère de la première, en ce qu'une copie de la base de données **2004** et du logiciel mettant en oeuvre la phase de prédiction **30, 30A** sont téléchargées dans la seconde organisation qui met en oeuvre à l'aide de l'ordinateur **2010** ou d'un serveur de calcul (non représenté) la totalité de la phase de prédiction **30, 30A.** Dans une troisième variante (fig. 22), la totalité des phases d'apprentissage **20, 20A** et de prédiction **30,30A** sont mises en oeuvre par une seule organisation **2006.**

G. Extension de l'enseignement du mode de réalisation

Il a été décrit un mode de réalisation de l'invention utilisant une PCR pour mesurer le profil génétique d'une souche bactérienne. En variante, ce profil est mesuré au moyen d'une puce à ADN ciblant lesdits marqueurs d'une manière connue en soi.

Il a été décrit un mode de réalisation dans lequel la base de données de génomes complets est indépendante, au moins dans sa première version, de l'écologie bactérienne de l'organisation mettant en oeuvre le typage pour ses propres besoins. En variante, cette base de données est constituée essentiellement des souches bactériennes en provenance uniquement de l'organisation. Une première version minimale de la base de données peut cependant être fournie à l'organisation puis complétée des souches de l'organisation.

Il a été décrit un mode de réalisation appliqué à une espèce bactérienne. L'invention s'applique également aux levures et aux champignons.

Il a été décrit un sérotypage. D'autres sous-types d'une espèce peuvent être caractérisés, comme par exemple leurs résistances et/ou sensibilités à des agents microbiens ou des groupes basés sur des séquences types.

Il a été décrit un taux d'assignation correcte ainsi qu'une précision, balancée ou non, comme critères de choix des hyperparamètres de la prédiction. D'autres critères sont possibles, comme par exemple la sensibilité et la spécificité ou un intervalle de confiance différent de 95%.

**Revendications**

**1.** Procédé *in vitro* de typage d'un microorganisme compris dans un échantillon, comprenant :

    a. la fourniture de moyens de stockages informatiques comprenant :

        - une base de données de profils génétiques de souches microbiennes appartenant à l'espèce microbienne dudit microorganisme, lesdits profils consistant en la présence ou l'absence d'un ensemble prédéfini de marqueurs génétiques ;
- un arbre phylogénétique de l'espèce dudit microorganisme, des assignations des profils génétiques de la base de données à des positions dans l'arbre phylogénétique, de fréquences de variation des profils génétiques des souches microbiennes de la base de données pour des niveaux prédéfinis de l'arbre phylogénique ;

b. la mesure du profil génétique du microorganisme ;
c. la détermination, mise en oeuvre par ordinateur, pour au moins un niveau de l'ensemble de niveaux prédéfinis de l'arbre phylogénétique :

- d'une probabilité de variation de chaque profil génétique de la base de données appartenant audit niveau par rapport au profil génétique mesuré, ladite probabilité de variation étant calculée en fonction des fréquences de variation audit niveau ;
- de l'appartenance du microorganisme à une subdivision dudit niveau si un premier critère de performance d'identification est atteint pour ladite subdivision, ledit premier critère étant atteint si au moins une probabilité de variation pour ladite subdivision est supérieure à un premier seuil prédéfini.

procédé dans lequel:

- l'arbre phylogénétique est construit à partir d'un core génome de l'espèce du microorganisme ; et
- l'ensemble prédéfini de marqueurs génétiques est choisi dans un génome accessoire de l'espèce du microorganisme.

2. Procédé selon la revendication 1, dans lequel si le premier critère de performance d'identification audit niveau n'est pas atteint alors l'étape c) est mise en oeuvre pour le niveau directement supérieur dans l'arbre phylogénique.

3. Procédé selon l'une quelconque des revendications précédentes, dans lequel le nombre de marqueurs des profils génétiques est inférieur à 50, et de préférence compris entre 16 et 30.

4. Procédé selon l'une quelconque des revendications précédentes, dans lequel :

- l'arbre phylogénétique est construit par l'application d'un clustering basé sur des distances génétiques;
- les distances génétiques décroissent depuis la racine vers les feuilles de l'arbre de sorte à définir un niveau dans l'arbre comprenant entre 150 et 350 différences dans le core génome.

5. Procédé selon l'une quelconque des revendications précédentes, dans lequel l'ensemble des marqueurs prédéfinis est sélectionné de manière à minimiser une entropie ou critère d'impureté dudit ensemble à un niveau de l'arbre phylogénique.

6. Procédé selon les revendications 4 et 5, dans lequel les marqueurs des profils génétiques sont sélectionnés de manière à minimiser une entropie ou un critère d'impureté au niveau comprenant entre 150 et 350 différences dans le core génome.

7. Procédé selon l'une quelconque des revendications précédentes, dans lequel le premier critère de performance d'indentification comprend en outre l'écart entre la probabilité de variation pour la subdivision et les probabilités de variation pour les autres subdivisions dudit niveau, le premier critère de performance d'identification étant atteint si ledit écart est supérieur à un second seuil prédéfini.

8. Procédé selon la revendication 7, dans lequel le second critère de performance d'identification comprend en outre l'écart entre la probabilité de variation pour ledit groupe et les probabilités de variation pour les autres groupes, le second critère de performance d'identification étant atteint si ledit écart est supérieur à un quatrième seuil prédéfini.

9. Procédé selon l'une quelconque des revendications précédentes, dans lequel la base de données comprend des couples de premier et second seuils, et pour chacun desdits seuils au moins un indicateur de performance d'identification, ledit indicateur de performance comprenant au moins un indicateur lié à la spécificité et/ou à la sensibilité du procédé d'identification.

**10.** Procédé selon la revendication 9, dans lequel l'indicateur de performance d'identification comprend un taux d'assignation correct et/ou une précision brute et/ou une précision balancée.

**11.** Procédé selon la revendication 9 ou 10, dans lequel les indicateurs de performance pour les couples de premier et second seuils sont affichés sur un écran, dans lequel un utilisateur sélectionne une valeur d'indicateur de performance d'identification affichée, et dans lequel le procédé fixe les premier et second seuils à leurs valeurs correspondant à la valeur d'indicateur de performance sélectionnée.

**12.** Procédé selon l'une quelconque des revendications précédentes, dans lequel la base de données comprend pour une partie au moins des profils génétiques, l'appartenance desdits profils génétiques à des groupes microbiens distincts, lesdits groupes distincts étant absents de l'arbre phylogénétique, le procédé comportant la détermination, mise en oeuvre par ordinateur :

- de probabilités de variation des profils génétiques appartenant auxdits groupes par rapport au profil génétique mesuré, lesdites probabilités de variation étant calculées en fonction des fréquences de variation à un niveau prédéfini de l'arbre phylogénétique ;
- de l'appartenance du microorganisme à l'un des groupes de microorganisme si subdivision dudit niveau si un second critère de performance d'identification est atteint pour ledit groupe, ledit second critère étant atteint si au moins une probabilité de variation pour ledit groupe est supérieure à un troisième seuil prédéfini.

**13.** Procédé selon la revendication 12, dans lequel le second critère de performance d'identification comprend en outre l'écart entre la probabilité de variation pour ledit groupe et les probabilités de variation pour les autres groupes, le second critère de performance d'identification étant atteint si ledit écart est supérieur à un quatrième seuil prédéfini.

**14.** Procédé selon la revendication 12 ou 13, dans lequel les groupes microbiens sont des sérotypes.

**15.** Procédé selon l'une quelconque des revendications précédentes, comprenant la recherche de causes racises lors d'une contamination d'un produit alimimentaire.

**16.** Procédé selon l'une quelconques des revendications précédentes, dans lequel la mesure du profil génétique du microorganisme est réalisé au moyen d'une amplification de séquences génétiques cibles sans mise en oeuvre de séquençage complet, en particulier une réaction de polymérisation en chaine.

**17.** Système de typage *in vitro* d'un microorganisme compris dans un échantillon, comprenant :

d. des moyens de stockages informatiques comprenant :

- une base de données de profils génétiques de souches microbiennes appartenant à l'espèce microbienne dudit microorganisme, lesdits profils consistant en la présence ou l'absence d'un ensemble prédéfini de marqueurs génétiques ;
- un arbre phylogénétique de l'espèce dudit microorganisme, des assignations des profils génétiques de la base de données à des positions dans l'arbre phylogénétique, de fréquences de variation des profils génétiques des souches microbiennes de la base de données pour des niveaux prédéfinis de l'arbre phylogénique ;

e. des moyens pour collecter et mémoriser une mesure du profil génétique du microorganisme ;
f. des moyens mis en oeuvre par ordinateur, de détermination pour au moins un niveau de l'ensemble de niveaux prédéfinis de l'arbre phylogénétique :

- d'une probabilité de variation de chaque profil génétique de la base de données appartenant audit niveau par rapport au profil génétique mesuré, ladite probabilité de variation étant calculée en fonction des fréquences de variation audit niveau ;
- de l'appartenance du microorganisme à une subdivision dudit niveau si un premier critère de performance d'identification est atteint pour ladite subdivision, ledit premier critère étant atteint si au moins une probabilité de variation pour ladite subdivision est supérieure à un premier seuil prédéfini.

**18.** Système selon la revendication 16, configuré pour mettre en oeuvre un procédé selon l'une quelconque des revendications 2 à 16.

**19.** Produit programme d'ordinateur enregistré sur un support informatique lisible par ordinateur comprenant des instructions pour l'exécution de l'étape c. d'un procédé selon l'une quelconque des revendications 1 à 16.

**20.** Produit programme d'ordinateur enregistré sur un support informatique lisible par ordinateur comprenant des instructions pour la construction d'un arbre phylogénétique en fonction d'un core-génome et des fréquences de variation d'un ensemble de marqueurs d'un génome accessoire à des niveaux de l'arbre phylogénétique.

**21.** Composition pour le typage *in vitro* de microorganismes compris dans un échantillon par amplification de séquences de nucléotides cibles, en particulier par une réaction de polymérisation en chaine, ladite composition comprenant un ensemble d'oligonucléotides pour l'amplification d'au moins l'ensemble de marqueurs génétiques obtenus selon la revendication 5 ou 6.

**22.** Procédé de préparation d'un échantillon biologique comprenant, ou susceptible de comprendre une souche bactérienne, en vue d'un typage de ladite souche par une réaction de polymérisation en chaine, comprenant la mise en contact dudit échantillon avec une composition selon la revendication 21.

**23.** Procédé selon la revendication 22, comprenant une lyse de l'échantillon issu de ladite mise en contact de manière à libérer du matériel génétique des bactéries de la souche bactérienne.

10 — Constitution ou complétion d'une base de données de génomes complets de souches bactériennes

20 — Phase d'apprentissage d'un arbre phylogénétique et de fréquences de variation d'un profil génétique

30 — Phase de prédiction : mesure du profil génétique d'une souche à tester et assignation de la souche à au moins un taxon en fonction des fréquences de variation et du profil mesuré

40 — Mise en œuvre de mesures épidémiologiques

**FIG. 1**

**FIG. 2**

Distance de partition

**FIG. 3**

Racine

Feuilles

Niveau de
l'arbre

Niveau

Racine

1

2

3

N

$T_1$ $T_2$ $T_3$ ... $T_{18}$ $T_{19}$ $T_{20}$

L

Feuilles

**FIG. 4**

N ---------------- $\bigcirc$ ---------------- T

N+1 ---- $\bigcirc$ $\bigcirc$ --- $\cdots$ --- $\bigcirc$ $\bigcirc$ ----

$T_1$ $T_2$ $T_{Q-1}$ $T_Q$

N ------------ $\{P_1, P_2, \dots, P_T\}$ -------

N+1 -- $\left\{P_1^{T_1}, P_2^{T_1}, \dots, P_{S_1}^{T_1}\right\}$ -- $\left\{P_1^{T_2}, P_2^{T_2}, \dots, P_{S_2}^{T_2}\right\}$ $\cdots$ $\left\{P_1^{T_{Q-1}}, P_2^{T_{Q-1}}, \dots, P_{S_{Q-1}}^{T_{Q-1}}\right\}$ $\left\{P_1^{T_Q}, P_2^{T_Q}, \dots, P_{S_Q}^{T_Q}\right\}$ ------

**FIG. 5**

50

| |
|---|
| Sélection d'amorces PCR pour le ciblage des marqueurs génétiques | 500 |
| Production des amorces | 502 |
| Production de kit PCR comprenant les amorces | 504 |

**FIG. 6**

**FIG. 7**

**FIG. 8**

20A

214 Profils génétiques

Sérotypes 224

226

$ST_1$  $ST_2$  $ST_3$  ...  $ST_{NS-1}$  $ST_{NS}$

228 Calcul de fréquences de variation des profils génétiques dans les sérotypes.

230 Fréq.de variation sérotypiques

**FIG. 9**

30A

300-306

228 Fréquences sérotypiques

224 Sérotypes

314 Calcul de probabilités de mutation du profil mesuré pour chaque sérotype

214 Profils génétiques

∃? un critère satisfaisant 316

Compte-rendu 318

**FIG. 10**

FIG. 11

Entropie de Shannon

FIG. 12

Nombre de marqueurs Mj

Fréquences de variation des 23 marqueurs dans les 24 niveaux de l'arbre phylogénétique

Abscisses : niveau dans l'arbre; Ordonnées: fréquences de variation

**FIG. 13**

Abscisses: taux moyen d'assignation sérotypique correcte; Ordonnées: précision balancée moyenne avec intervalle de confiance à 95%

**FIG. 14**

Abscisses: taux moyen d'assignation phylogénétique correcte: Ordonnées: précision moyenne avec un intervalle de confiance à 95%

**FIG. 15**

Entropie de Shannon

FIG. 16

Nombre de marqueurs Mj

EP 4 471 784 A1

Fréquences de variation des 23 marqueurs dans les 24 niveaux de l'arbre phylogénétique

FIG. 17

Abscisses : niveau dans l'arbre; Ordonnées: fréquences de variation

28

Abscisses: taux moyen d'assignation sérotypique correcte; Ordonnées: précision balancée moyenne avec intervalle de confiance à 95%

**FIG. 18**

**FIG. 19**

Abscisses: taux moyen d'assignation phylogénétique correcte: Ordonnées: précision moyenne avec un intervalle de confiance à 95%

FIG. 20

2000

2002

2004

2012

2006

2008

2010

FIG. 21

2000

2002

2004

2012

2006

2008

2010

2004

FIG. 22

Europäisches
Patentamt

European
Patent Office

Office européen
des brevets

**RAPPORT DE RECHERCHE EUROPEENNE**

Numéro de la demande

**EP 23 17 6319**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | PINTO M ET AL: "Insights into the population structure and pan-genome of Haemophilus influenzae", INFECTION , GENETICS AND EVOLUTION, ELSEVIER, AMSTERDAM, NL, vol. 67, 31 octobre 2018 (2018-10-31), pages 126-135, XP085569133, ISSN: 1567-1348, DOI: 10.1016/J.MEEGID.2018.10.025 * le document en entier * ----- | 1-20 | INV. G16B10/00 G16B40/20 G16B20/20 G16B20/40 G16B30/10 |
| A | JAGADEESAN BALAMURUGAN ET AL: "The use of next generation sequencing for improving food safety: Translation into practice", FOOD MICROBIOLOGY, ACADEMIC PRESS LTD, LONDON, GB, vol. 79, 1 juin 2019 (2019-06-01), pages 96-115, XP085574633, ISSN: 0740-0020, DOI: 10.1016/J.FM.2018.11.005 [extrait le 2018-11-17] * le document en entier * ----- | 1-20 | |
| A | TODD J TREANGEN ET AL: "The Harvest suite for rapid core-genome alignment and visualization of thousands of intraspecific microbial genomes", GENOME BIOLOGY, BIOMED CENTRAL LTD, vol. 15, no. 11, 19 novembre 2014 (2014-11-19), page 524, XP021206431, ISSN: 1465-6906, DOI: 10.1186/S13059-014-0524-X * le document en entier * ----- -/-- | 1-20 | DOMAINES TECHNIQUES RECHERCHES (IPC) G16B |

Le présent rapport a été établi pour toutes les revendications

| Lieu de la recherche | Date d'achèvement de la recherche | Examinateur |
|---|---|---|
| Munich | 22 janvier 2024 | Schechner-Resom, G |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
........................................................................
& : membre de la même famille, document correspondant

EPO FORM 1503 03.82 (P04C02)

page 1 de 2

Europäisches
Patentamt

European
Patent Office

Office européen
des brevets

## RAPPORT DE RECHERCHE EUROPEENNE

Numéro de la demande

EP 23 17 6319

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2018/365375 A1 (FLYGARE STEVEN [US] ET AL) 20 décembre 2018 (2018-12-20) * alinéa [0070] – alinéa [0110] * * figures 1-3 * | 1-20 | |
| A | WO 2022/159838 A1 (IDBYDNA INC [US]; XIE HENG [US] ET AL.) 28 juillet 2022 (2022-07-28) * alinéa [0002] – alinéa [0072] * | 1-20 | |
| X | US 2016/114322 A1 (ISMAGILOV RUSTEM F [US] ET AL) 28 avril 2016 (2016-04-28) * abrégé * * alinéas [0004], [0095] – alinéa [0129] * * figure 20 * | 21-23 | |
| A | US 10 865 447 B2 (IMMUNEXPRESS PTY LTD [AU]) 15 décembre 2020 (2020-12-15) * abrégé * * colonne 57, ligne 37 – colonne 59, ligne 33 * * colonne 95, ligne 64 – colonne 100, ligne 49 * | 21-23 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 22 janvier 2024 | Schechner-Resom, G |

EPO FORM 1503 03.82 (P04C02)

page 2 de 2

Europäisches
Patentamt

European
Patent Office

Office européen
des brevets

Numéro de la demande

EP 23 17 6319

## REVENDICATIONS DONNANT LIEU AU PAIEMENT DE TAXES

La présente demande de brevet européen comportait lors de son dépôt les revendications dont le paiement était dû.

☐ Une partie seulement des taxes de revendication ayant été acquittée dans les délais prescrits, le présent rapport de recherche européenne a été établi pour les revendications pour lesquelles aucun paiement n'était dû ainsi que pour celles dont les taxes de revendication ont été acquittées, à savoir les revendication(s):

☐ Aucune taxe de revendication n'ayant été acquittée dans les délais prescrits, le présent rapport de recherche européenne a été établi pour les revendications pour lesquelles aucun paiement n'était dû.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

**voir feuille supplémentaire B**

☒ Toutes les nouvelles taxes de recherche ayant été acquittées dans les délais impartis, le présent rapport de recherche européenne a été établi pour toutes les revendications.

☐ Comme toutes les recherches portant sur les revendications qui s'y prêtaient ont pu être effectuées sans effort particulier justifiant une taxe additionnelle, la division de la recherche n'a sollicité le paiement d'aucune taxe de cette nature.

☐ Une partie seulement des nouvelles taxes de recherche ayant été acquittée dans les délais impartis, le présent rapport de recherche européenne a été établi pour les parties qui se rapportent aux inventions pour lesquelles les taxes de recherche ont été acquittées, à savoir les revendications:

☐ Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications, à savoir les revendications:

☐ Le present rapport supplémentaire de recherche européenne a été établi pour les parties de la demande de brevet européen qui se rapportent a l'invention mentionée en premier lieu dans le revendications (Règle 164 (1) CBE)

Europäisches
Patentamt

European
Patent Office

Office européen
des brevets

## ABSENCE D'UNITÉ D'INVENTION
## FEUILLE SUPPLÉMENTAIRE B

Numéro de la demande

EP 23 17 6319

La division de la recherche estime que la présente demande de brevet européen ne satisfait pas à l'exigence relative à l'unité d'invention et concerne plusieurs inventions ou pluralités d'inventions, à savoir :

    1. revendications: 1-20

        identification d'un microorganisme par une méthode mise en oeuvre par ordinateur
        ---

    2. revendications: 21-23

        préparation d'un échantillon en vue d'un séquençage
        ---

EPO FORM P0402

**ANNEXE AU RAPPORT DE RECHERCHE EUROPEENNE
RELATIF A LA DEMANDE DE BREVET EUROPEEN NO.**

**EP 23 17 6319**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**22-01-2024**

| Document brevet cité au rapport de recherche | | | Date de publication | Membre(s) de la famille de brevet(s) | | Date de publication |
|---|---|---|---|---|---|---|
| US 2018365375 | A1 | | 20-12-2018 | AU | 2016253004 A1 | 07-09-2017 |
| | | | | AU | 2023200050 A1 | 09-02-2023 |
| | | | | CA | 2977548 A1 | 27-10-2016 |
| | | | | CN | 107532332 A | 02-01-2018 |
| | | | | CN | 115206436 A | 18-10-2022 |
| | | | | EP | 3286359 A2 | 28-02-2018 |
| | | | | IL | 281001 A | 29-04-2021 |
| | | | | US | 2018365375 A1 | 20-12-2018 |
| | | | | US | 2023055403 A1 | 23-02-2023 |
| | | | | WO | 2016172643 A2 | 27-10-2016 |
| | | | | ZA | 201707500 B | 25-10-2023 |
| WO 2022159838 | A1 | | 28-07-2022 | CN | 116802313 A | 22-09-2023 |
| | | | | EP | 4281582 A1 | 29-11-2023 |
| | | | | WO | 2022159838 A1 | 28-07-2022 |
| US 2016114322 | A1 | | 28-04-2016 | AU | 2014253749 A1 | 26-11-2015 |
| | | | | CA | 2918388 A1 | 23-10-2014 |
| | | | | CN | 105745172 A | 06-07-2016 |
| | | | | EP | 2986554 A1 | 24-02-2016 |
| | | | | JP | 6533516 B2 | 19-06-2019 |
| | | | | JP | 2016518834 A | 30-06-2016 |
| | | | | KR | 20160087749 A | 22-07-2016 |
| | | | | SG | 10201800317P A | 27-02-2018 |
| | | | | SG | 11201508618R A | 27-11-2015 |
| | | | | US | 2016114322 A1 | 28-04-2016 |
| | | | | US | 2019336969 A1 | 07-11-2019 |
| | | | | WO | 2014172688 A1 | 23-10-2014 |
| | | | | ZA | 201508323 B | 27-03-2019 |
| US 10865447 | B2 | | 15-12-2020 | AU | 2015213486 A1 | 02-06-2016 |
| | | | | CA | 2930925 A1 | 13-08-2015 |
| | | | | CN | 105981026 A | 28-09-2016 |
| | | | | DK | 3103046 T3 | 02-06-2020 |
| | | | | EP | 3103046 A1 | 14-12-2016 |
| | | | | JP | 2017512304 A | 18-05-2017 |
| | | | | US | 2015218640 A1 | 06-08-2015 |
| | | | | US | 2015259746 A1 | 17-09-2015 |
| | | | | US | 2017191129 A1 | 06-07-2017 |
| | | | | US | 2022325348 A1 | 13-10-2022 |
| | | | | WO | 2015117204 A1 | 13-08-2015 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **HU et al.** Phylogenetics Algorithms and Applications. *Ambient Communications and Computer Systems*, 2019
- **SHAKTAWAT et al.** A Review: Phylogeny Construction Methods. *International Journal of Emerging Science and Engineering*, 2019
- **JIN XIONG**. Essential Bioinformatics. Cambridge University, 2011
- **SCHÜRCH, A. et al.** Whole genome sequencing options for bacterial strain typing and épidémiologie analysis based on single nucléotide polymorphism versus gene-by-gene-based approaches. *Clin. Microbiol. Infect.*, 2018
- **SILVA M et al.** chewBBACA: a complété suite for gene-by-gene schéma création and strain identification.. *Microb. Genom.*, 2018
- **ZHOU et al.** HierCC: a multi-level clustering scheme for population assignments based on core genome MLST. *Bioinformatics*, 2021, https://pathogen.watch